Europäisches Patentamt

European Patent Office

Office européen des brevets

(19)

(11) Veröffentlichungsnummer: **0 283 706 B1**

# (12) EUROPÄISCHE PATENTSCHRIFT

(45) Veröffentlichungstag der Patentschrift: 07.08.91

(51) Int. Cl.⁵: **A61F 2/36**

(21) Anmeldenummer: **88102112.5**

(22) Anmeldetag: **12.02.88**

(54) **Hüftprothese.**

(30) Priorität: **09.03.87 DE 8703491 U**

(43) Veröffentlichungstag der Anmeldung:
**28.09.88 Patentblatt 88/39**

(45) Bekanntmachung des Hinweises auf die
Patenterteilung:
**07.08.91 Patentblatt 91/32**

(84) Benannte Vertragsstaaten:
**AT BE CH DE ES FR GB IT LI LU NL SE**

(56) Entgegenhaltungen:
EP-A- 0 145 641      EP-A- 0 181 586
DE-A- 3 205 577      DE-A- 3 336 005
SU-A- 762 871        US-A- 4 077 070
US-A- 4 227 518

(73) Patentinhaber: **Waldemar Link GmbH & Co**
**Barkhausenweg 10**
**W-2000 Hamburg 63(DE)**

(72) Erfinder: **Keller, Arnold**
**An der Nahefurth 5**
**W-2061 Kayhude(DE)**

(74) Vertreter: **Glawe, Delfs, Moll & Partner Patent-**
**anwälte**
**Postfach 26 01 62 Liebherrstrasse 20**
**W-8000 München 26(DE)**

**Beschreibung**

Die Erfindung betrifft eine Hüftprothese gemäß dem Oberbegriff des Anspruchs 1. Eine derartige Prothese ist aus der EP-A-0181586 bekannt.

Die US-A-4227518 offenbart eine Femurprothese mit einem U-förmigen Stützteil zur Abstützung auf einer Resektionsfläche des unteren Abschnitts des Oberschenkelknochens. Dieses U-förmige Stützteil wird 4-6 Monate nach der Implantation der Femurprothese wieder aus dem Knochen entfernt, was sich störend auf den Heilungsprozeß auswirkt.

Nach Implantation einer Hüftprothese kann es zu einer Destruktion des obersten Oberschenkelabschnitts kommen, die sich in einer äußerst dünnen oder sogar teilweise fehlenden Kortikalis zeigt. Muß in einem solchen Zustand eine Re-Operation vorgenommen werden, so kann dieser Abschnitt zur Abstützung der neuen Prothese nicht herangezogen werden. Für diesen Fall stellt der Stand der Technik zwei Prothesenversionen zur Verfügung. Die eine dieser Versionen beruht auf dem Ersatz des degenerierten, oberen Abschnitts des Oberschenkelknochens, der entfernt und durch eine Prothese ersetzt wird. Diese stützt sich auf der Resektionsebene ab und ihr Schaft wird im distal verbliebenen Teil des Knochens verankert. Der Nachteil dieser Version besteht im Verlust des natürlichen Muskelansatzes im Trochanterbereich und in der Einschränkung weiterer prothetischer Maßnahmen in der Folgezeit. Nicht selten entsteht dadurch ein totaler Verlust des Oberschenkelknochens. Die zweite, häufiger verwendete Version besteht in einer Prothese mit einem langen Schaft, der über den degenerierten Abschnitt des Oberschenkelknochens hinabreicht und tief im distalen Abschnitt des Knochens einzementiert wird. Nachteilig ist dabei, daß im geschwächten Abschnitt des Oberschenkelabschnitts für die Halsauflage der Prothese in der Regel keine sichere Abstützung am Knochen erreicht werden kann. Damit besteht die Gefahr des Einsinkens der Prothese in den Oberschenkelknochen. Durch die mangelnde Abstützung des oberen Prothesenteils wird dieser erhöhter Schwingbelastung und damit erhöhter Bruchgefahr unterworfen. Das totale Ausfüllen des degenerierten Knochenabschnitts mit Zement provoziert aus biomechanischen und biologischen Gründen erwiesenermaßen den totalen Ausfall der restlich verbliebenen, dünnen Kortikalis.

Eine Regeneration des geschwächten Knochenabschnitts kann nicht mit Zement, sondern nur mit zementloser Implantationstechnik erreicht werden. Sie ist aber nur dann zu erwarten, wenn Bewegungsruhe zwischen Prothese und Knochen besteht, die eine stabile Prothesenverankerung im unteren Knochenabschnitt unter Verhinderung des Einsinkens der Prothese voraussetzt.

Der Erfindung liegt die Aufgabe zugrunde, eine Prothese der Eingangs genannten Art zu schaffen, die diesen Zielen besser gerecht wird.

Die erfindungsgemäße Lösung besteht in den Kennzeichnenden Merkmalen des Anspruchs 1. Der zwischen den Stützteilen gebildete Zwischenraum ermöglicht es, daß eine Knochenverbindung zwischen den unterhalb und oberhalb der Stützteile befindlichen Abschnitte des Oberschenkelknochens verbleibt oder wieder entsteht. Die Erfindung schafft somit die Möglichkeit, daß die Prothese sich in einem mittleren Teil des Oberschenkelknochens abstützt, ohne zur vollständigen Resektion des oberen Abschnitts zu zwingen. Der ausgedünnte, obere Abschnitt kann vielmehr mit natürlichem, homologen bzw. autologen Knochenmaterial aufgefüllt und dadurch gestärkt werden, ohne daß der Halt der Prothese auf diesen Knochenabschnitt angewiesen ist.

Die Abstützung der Prothese braucht nicht ausschließlich auf den mit Zwischenraum voneinander in den Knochen eingreifenden Stützteilen zu beruhen; es kann vielmehr auch eine ringförmige Abstützfläche Verwendung finden, deren Außendurchmesser geringer ist als der Außendurchmesser des Knochens an der betreffenden Stelle. In vielen Fällen genügt es, wenn die mit Zwischenraum voneinander in den Knochen eingreifenden Stützteile lediglich in einer Ebene liegen; jedoch ist auch die Verwendung von Stützteilen in mehreren Ebenen möglich, beispielsweise mittels Vorsprüngen, die igelartig an einem oder mehreren Ringteilen angeordnet sind.

Für die Implantation eines Rings, der Stützteile in einer Ebene trägt, bietet sich die Möglichkeit an, den Ring nach vollständiger Durchtrennung des Oberschenkelknochens an der betreffenden Stelle einzubringen. Danach wird dann der Prothesenschaft in den Markkanal eingeschlagen. Dabei ist der Ring im wesentlichen innerhalb des Markraumes positioniert, so daß anschließend - ähnlich wie bei einer Fraktur - ein lineares Durchwachsen der Kortikalis von distal nach proximal ermöglicht wird. Statt dessen kann es in manchen Fällen auch möglich sein, zunächst den Schaft einzubringen und die Stützteile von außen her durch den Knochen einzusetzen und mit dem Prothesenschaft zu verbinden.

Wenn die Stützteile an einem gesondert von dem Schaft einbringbaren Teil angeordnet sind, weist der Schaft zweckmäßigerweise eine Anschlagfläche auf, mit der er sich seinerseits daran abstützt. Zur Einstellung der richtigen Lage der Prothese können Zwischenringe vorgesehen sein, die zwischen die Anschlagfläche des Schafts und den die Stützteile tragenden Teil eingesetzt werden können. Ferner kann zur Einstellung vorgesehen sein, daß zwischen einem dem Prothesenkopf tragenden Prothesenteil und dem die Stützteile bildenden oder tragenden Prothesenteil Zwischenstücke einstellbarer Länge angeordnet werden können.

Die Erfindung wird im folgenden näher unter Bezugnahme auf die Zeichnung erläutert, die ein vorteilhaftes Ausführungsbeispiel veranschaulicht. Darin zeigen:

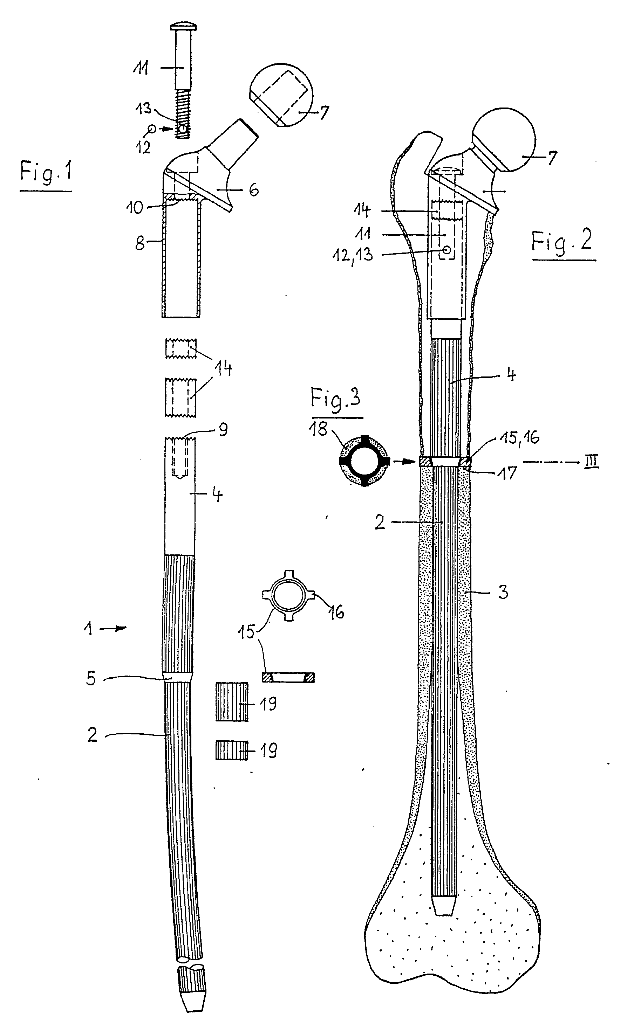

Fig. 1 die Prothese im auseinander gezogenen Zustand ihrer Einzelteile,

Fig. 2 die Prothese im eingesetzten Zustand und

Fig. 3 einen Querschnitt durch Fig. 2 in der Ebene III

Der Prothesenschaft 1 besteht aus einem unteren Abschnitt 2, der in dem mittleren bis unteren Abschnitt des Oberschenkelknochens 3 einzubringen ist, und einem oberen Abschnitt 4, der einen etwas größeren Durchmesser als der untere Abschnitt 2 aufweist, so daß an der Übergangsstelle durch einen Durchmessersprung eine Anschlagfläche 5 gebildet wird. Der Abschnitt 2 und ggf. teilweise der Abschnitt 1 können Längsriefen zwecks großflächigerer Verbindung mit dem Knochenmaterial aufweisen. Der obere Abschnitt 4 des Schafts ist in beliebiger, bekannter Weise mit dem Halsteil 6 lösbar verbunden, der den Prothesenkopf 7 trägt. Im dargestellten Fall wird die Verbindung dadurch bewerkstelligt, daß der Halsteil 6 eine Hülse 8 aufweist, die den oberen Schaftabschnitt 4 passend umgibt zwecks besserer Übertragung der Biegemomente. Die einander gegenüber stehenden Stirnflächen 9 des Prothesenschafts und 10 des Hülsenbodens weisen eine ineinander greifende, radiale Zahnung auf, die eine drehsichere Verbindung mittels der Zugschraube 11 in unterschiedlichen Winkelstellungen zwecks Einstellung der gewünschten Anteversion ermöglichen. Die Zugschraube 11 kann mittels einer bekannten Sicherung 12 in einer Bohrung 13 gegen ungewollte Verdrehung gesichert werden. Zwischenstücke 14 erlauben die Einstellung eines gewünschten Abstands zwischen dem Gelenkkopf 7 und der Anschlagfläche 5.

Im implantierten Zustand stützt sich die Prothese über die Anschlagfläche 5 an einem Stützring 15 ab, der eine zur Anschlagfläche 5 passende Bohrung aufweist und von den mehrere Stützteile 16 als radiale Vorsprünge ausgehen. Der Außendurchmesser des die Stützteile 16 tragenden Stützrings 15 ist größer als der Innendurchmesser des Markkanals aber kleiner als der Außendurchmesser des Oberschenkelknochens. Sowohl der Stützring als auch die Vorsprünge können sich daher auf der Resektionsfläche 17 des Oberschenkelknochens abstützen. Gleichzeitig bleibt zwischen den Stützvorsprüngen im Querschnittsbereich des Knochens hinreichender Zwischenraum, durch den Knochenmaterial 18 den oberen und den unteren Abschnitt des Oberschenkelknochens verbinden kann. Es sind Zwischenringe 19 zur Einstellung des Abstands zwischen der Anschlagfläche 5 und dem Stützring 15 vorgesehen.

Operativ wird in der Weise vorgegangen, daß in einer Höhe, in der die Kortikalis des Oberschenkelknochens ausreichend stark ist, der Oberschenkelknochen durchtrennt wird. An dieser Stelle wird der Stützring 15 zwischengelegt. Danach wird von proximal der Prothesenschaft 1 in den Markkanal eingeschlagen.

Der obere, ausgedünnte Oberschenkelabschnitt wird mit natürlichem, homologen bzw. autologen Knochenmaterial aufgefüllt und der Halsteil 6 auf den Prothesenschaft aufgesteckt und in der gewünschten Anteversionslage befestigt. Die Zwischenstücke 14 bzw. 19 ermöglichen dabei eine richtige Längeneinstellung. Es kann vorgesehen sein, daß der Ringteil 15 des Stützrings überwiegend oder ausschließlich innerhalb des Markkanals liegt, so daß zwischen den Stützvorsprüngen 16 ein maximaler Querschnittsbereich für das lineare Durchwachsen der Kortikalis verbleibt.

Durch die erfindungsgemäße erreichbare Knochenrekonstruktion werden in der Folge möglicherweise notwendige Maßnahmen und Re-Operationen wesentlich begünstigt und die Gefahr des totalen Verlusts des Oberschenkelknochens wesentlich eingeschränkt.

**Patentansprüche**

1. Hüftprothese für einen Oberschenkelknochen (3), der einen unteren belastbaren Abschnitt und einen oberen, zu erhaltenden, geschwächten Abschnitt aufweist, wobei die Hüftprothese einen Schaft (1) besitzt, der in dem unteren Abschnitt des Oberschenkelknochens (3) zu verankern ist, dadurch gekennzeichnet, daß ein mit dem Schaft (1) der Prothese verbindbarer, gesonderter Teil (15) vorgesehen ist, von dem eine Mehrzahl von in den Knochen eingreifenden Stützteilen (16) sternartig ausgeht, die eine quer zur Schaftrichtung verlaufende Stützfläche zur Auflage auf einer Resektionsfläches (17) des unteren Abschnitts des Oberschenkelknochens (3) bilden und die miteinander Zwischenräume zur Aufnahme einer Knochenverbindung zwischen dem unteren und dem oberen Abschnitt des Oberschenkelknochens einschließen.

2. Prothese nach Anspruch 1, dadurch gekennzeichnet, daß die Stützteile (16) sternartig von einem Ring (15), einer Hülse oder dergleichen ausgehen.

**3.** Prothese nach Anspruch 2, dadurch gekennzeichnet, daß der Schaft (1) eine mit dem Ring (15), der Hülse oder dergleichen zusammenwirkende Anschlagfläche (5) aufweist.

**4.** Prothese nach Anspruch 3, dadurch gekennzeichnet, daß zwischen der Anschlagfläche (5) und dem Ring (15) der Hülse oder dergleichen einsetzbare Zwischenringe (19) vorgesehen sind.

**5.** Prothese nach einem der Ansprüche 1 bis 4, dadurch gekennzeichnet, daß der den Prothesenkopf (7) tragende Prothesenteil (6) mit dem die Stützteile (16) bildenden oder tragenden Prothesenteil (1) lösbar über Zwischenstücke (14) einstellbarer Länge verbunden ist.

## Claims

**1.** A hip prosthesis for a thigh bone (3), which has a lower load-bearing portion and a weakened upper portion to be maintained, wherein the hip prosthesis has a shaft (1) which is to be anchored in the lower portion of the thigh bone (3), characterised in that a separate part (15) is provided which can be joined to the prosthesis shaft (1) and from which extend radially a plurality of support members (16) engaging into the bone, which support members form a support surface extending transversely to the shaft direction to bear against a resection surface (17) of the lower portion of the thigh bone (3) and which together enclose spaces for accommodating a bone connection between the lower portion and upper portion of the thigh bone.

**2.** A prosthesis according to Claim 1, characterised in that the support members (16) extend radially from a ring (15), a collar or the like.

**3.** A prosthesis according to Claim 2, characterised in that the shaft (1) has an abutment surface (5) co-operating with the ring (15), collar or the like.

**4.** A prosthesis according to Claim 3, characterised in that intermediate rings (19) are provided which can be inserted between the abutment surface (5) and the ring (15), collar or the like.

**5.** A prosthesis according to any one of Claims 1 to 4, characterised in that the prosthesis part (6) carrying the prosthesis head (7) is detachably connected to the prosthesis part (1) forming or carrying the support members (16) via intermediate members (14) of adjustable length.

## Revendications

**1.** Prothèse de hanche pour un fémur (3) qui présente un segment inférieur capable de porter la charge et un segment supérieur affaibli à conserver, la prothèse de hanche comportant une tige (1) destinée à être ancrée dans le segment inférieur du fémur (3), caractérisée en ce qu'il est prévu une pièce séparée (15) qui peut être fixée à la tige (1) de la prothèse et sur laquelle font saillie en étoile plusieurs éléments d'appui (16) qui s'insèrent dans l'os, constituent une surface d'appui s'étendant perpendiculairement à la direction de la tige et destinée à prendre appui sur une surface de résection (17) du segment inférieur du fémur (3) et délimitent entre eux des intervalles destinés à recevoir une jonction osseuse entre les segments inférieur et supérieur du fémur.

**2.** Prothèse selon la revendication 1, caractérisée en ce que les éléments d'appui (16) font saillie en étoile sur un anneau (15), une douille ou une pièce similaire.

**3.** Prothèse selon la revendication 2, caractérisée en ce que la tige (1) présente une surface de butée (5) qui coopère avec l'anneau (15), la douille ou la pièce similaire.

**4.** Prothèse selon la revendication 3, caractérisée en ce qu'il est prévu des anneaux intermédiaires (19) qui peuvent être interposés entre la surface de butée (5) et l'anneau (15), la douille ou la pièce similaire.

**5.** Prothèse selon l'une quelconque des revendications 1 à 4, caractérisée en ce que la partie (6) de la prothèse qui porte la tête (7) de la prothèse est fixée de façon détachable, avec interposition de pièces intermédiaires (14) de longueur réglable, à la partie (1) de la prothèse qui forme ou qui porte les éléments d'appui (16).

Fig.1

Fig.2

Fig.3